# EUROPEAN PATENT SPECIFICATION

(11) **EP 4 164 780 B1**
(45) Date of publication and mention of the grant of the patent: **01.10.2025**
(21) Application number: 21737296.0
(22) Date of filing: 11.06.2021
(51) Int. Cl.: B01F 25/433, B01F 25/50, B01F 25/51, C07K 1/14, C12M 1/00

(54) **PLUG FLOW REACTOR AND METHOD FOR PROVIDING A HOMOGENEOUS FEED STREAM WITHIN THE PLUG FLOW REACTOR**
PLUG FLOW REAKTOR UND VERFAHREN ZUR BEREITSTELLUNG EINES HOMOGENEN EINSATZSTROMS IN EINEM PLUG FLOW REAKTOR
RÉACTEUR EN ÉCOULEMENT PISTON ET PROCÉDÉ DE FOURNITURE D'UN FLUX D'ALIMENTATION HOMOGÈNE À L'INTÉRIEUR D'UN RÉACTEUR EN ÉCOULEMENT PISTON

(30) Priority: 16.06.2020 US 202063039541 P
(43) Date of publication of application: 19.04.2023
(73) Proprietor: Boehringer Ingelheim International GmbH, 55216 Ingelheim am Rhein (DE)
(72) Inventor: KWONG, Aaron Thomas, Ridgefield, Connecticut 06877-0368 (US); BROWN, Matthew Robert, Ridgefield, Connecticut 06877-0368 (US); GODFREY, Scott A., Ridgefield, Connecticut 06877-0368 (US)
(74) Representative: Höfer, Friederike
(86) International application number: PCT/US2021/036917
(87) International publication number: WO 2021/257387

(56) References cited:
- EP-A1- 1 803 497
- WO-A1-2020/076681

## Description

### FIELD OF THE INVENTION

The present disclosure generally relates to the field of biotechnological production, more specifically to continuous bioprocessing and to a device and a method for an in-line homogenization of a feed stream within a plug flow reactor (PFR).

### BACKGROUND OF THE INVENTION

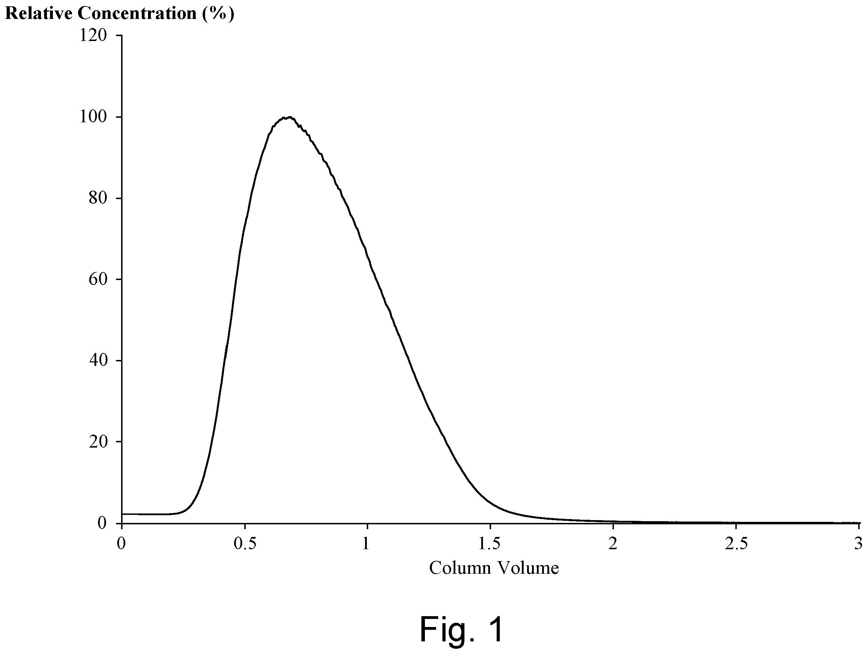

In continuous bioprocessing, at least one unit operating within the manufacturing cascade receives a continuous input of a material of interest without interruption. An essential element for continuous bioprocessing is simultaneous or continuous viral inactivation (CVI), a necessary step after the appropriate chromatographic purification steps that may include bind-and-elute column chromatography such as Protein A (ProA) chromatography. For example, the product or material of interest of a variable concentration may be eluted from a ProA column in a manner represented by an asymmetric Gaussian curve primarily based on the inherent properties of the specific bind-and-elute column (Fig. 1). In the next step of the bioprocess after bind-and-elute column chromatography, attempts to homogenize the product feed stream before further analysis, either use a surge vessel such as a tank or bag; direct-transfer tubing; or alternative means such as recycling the feed stream. The following prior art publications are examples of the state of the art, which contains inherent deficiencies and drawbacks.

WO 2014/004103 teaches a system design and method for the in-line addition of a viral-inactivation reagent, such as an acid or a detergent, to a composition including a biological product continuously. This system design and method uses one or more in-line static mixers with mixing elements to combine the biological product with a viral-inactivation reagent during flow from a first unit operation to a second unit operation.

WO 2015/158776 teaches a system design and method that uses a surge vessel to combine a viral-inactivation reagent, such as an acid or a detergent, to a composition including a biological product continuously, to inactivate viruses that may be present in the composition. In particular, blending multiple column elution cycles makes troubleshooting difficult because if a deviation from the normal operation occurred in one of the cycles, the blending process obscures the location of the deviant product. Also, surge vessels or an open circuit homogenization loops make system sanitization difficult because product retention in the system is not easily addressed.

US 2018/0117495 A1 teaches the process of continuous purification of a target biological molecule in which viruses are inactivated in-line within the continuous purification system by a method of in-line blending ProA elution peaks within an open circuit flow path where previously eluted peaks are blended backward with currently eluting peaks. This technology utilizes an open circuit homogenization loop to direct product flow through an in inlet and an outlet during the mixing. This methodology minimally dampens the ProA peaks as they move through the system and aims to redistribute the acid used to lower the pH more evenly. The relative flow rates utilized in this system design and method are not ideal for scaling up the process; the flow rate during the redistribution or recycle step is 90 times faster than the incubation step.
WO 2020/076681 A1 discloses a viral inactivation device comprising: at least one continuous viral inactivation reactor including an inlet, an outlet, and a tubular flow path comprising a set of alternating turns that form a serpentine pattern between the inlet and the outlet.
In EP 1 803 497 A1 there are provided methods and systems for generating nanoparticles from an inorganic precursor compound using a hydrothermal process within at least one continuously stirred tank reactor (CSTR) or plug flow reactor (PFR) (102) maintained at an elevated temperature and an elevated pressure and a treatment vessel in which this reaction solution can be applied to one or more catalyst substrates.EP 1803 497 A1 discloses a device suitable for in-line homogenization as per the preamble of claim 1.

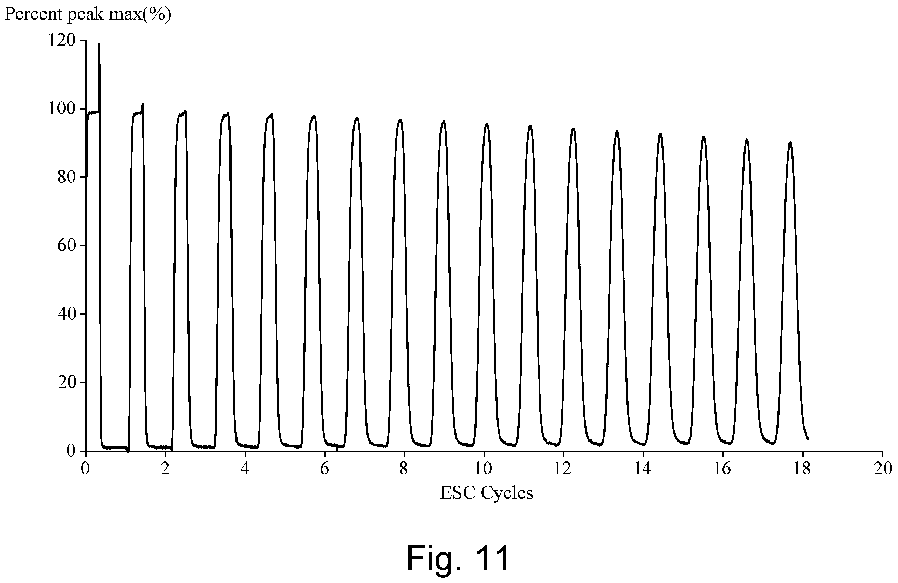

Additional attempts to avoid surge vessels implement a tank-less hold that captures one elution cycle for a subsequent transfer of the non-uniform feedstream to the next step in the process to a unit in which the non-uniform feed stream (with a protein or product of interest) is replaced with a non-product containing fluid thereby voiding the product to prepare for the cleaning/sanitization process required before the next cycle is captured. This strategy, however, is typically unable to produce homogenization because after repeated circulations, the feed stream remains non-uniform (Figure 11). It is well-known in the art, that a non-uniform feed stream composition results in mutable viscosity and a variable protein concentration, both of which negatively affect the downstream CVI step. Therefore, there is a need for a device or method to reduce or eliminate these complexities.

### SUMMARY OF THE INVENTION

The present invention solves the above problems with the methods and devices disclosed herein, which results in faster and better homogenization of a non-uniform feed stream (e.g., from a ProA column) than the existing art.

In one or more aspects, embodiments, the invention is directed to a device for an in-line homogenization of a non-uniform feed stream. In our system, the eluted product, which will be defined as the feed stream which contains at least the product of interest, is first stored within a plug flow reactor (PFR) as a non-uniform feed stream in which the concentration of the stream is variable in that it has a maximum and minimum concentration. The contained non-uniform feed stream is then circulated until the difference between the maximum and the minimum concentration is reduced to a sufficient range, the maximum is sufficiently reduced, or the minimum is sufficiently increased.

In one embodiment, the device for in-line homogenization of a non-uniform feed stream as per the subject-matter of claim 1 comprises a flow path comprising: (a) a plug flow reactor (PFR); (b) a pump for circulating the non-uniform feed stream through the flow path, and (c) a bypass line for diverting a percentage of the non-uniform feed stream from a first location inside the PFR to a second location within the flow path. In a preferred embodiment the non-uniform feed stream comprises a protein or product of interest.

In another embodiment, the device is in-line placed between a first and a second process step. In an aspect, the first process step is a bind and elute column (Protein A) chromatography. In one aspect, the second process step is a virus inactivation step. In another aspect of the device, the flow path is a closed-circuit flow path. In yet another aspect, the device is tank-less.

In one embodiment, the PFR comprises a tubing assembly packed in at least one chamber. The device may also include a bypass line as an extension of the flow path that fluidically connects to locations within the flow path. In one aspect, the second location is downstream from the first location. In another aspect, the second location is upstream from the first location. Alternatively, the second location is placed inside the PFR downstream or upstream from the first location. In one aspect of the device, the pump is selected from a group consisting of a centrifugal pump and /or a positive displacement pump. The device may further comprise a second pump in the flow path, wherein the second pump modulates the non-uniform feedstream flow in the bypass line. In another aspect, the second pump is a pump selected from the group consisting of a centrifugal pump and/or a positive displacement pump. In one aspect, the non-uniform feed stream flow in the bypass line is co-current or counter-current to the non-uniform feed stream flow in the flow path.

In another embodiment, a method for in-line homogenization of a non-uniform feed stream as per the subject-matter of claim 7 is provided, the method includes: (a) providing a non-uniform feed stream, (b) flowing the non-uniform feed stream through a flow path comprising a PFR, a bypass line, and a pump; (c) diverting a percentage of the non-uniform feed stream from a first location inside the PFR to a second location within the flow path; (d) circulating the non-uniform feed stream through the flow path; and (e) homogenizing the non-uniform feed stream. In a preferred embodiment this method comprises a protein or product of interest In one aspect of the method, the flow path is in-line between a first and a second process step. In an alternative aspect, the first process step is a Protein A (ProA) column chromatography. The second process step in the method can be a virus inactivation step. In an embodiment, the method is directed to virus inactivation.

In one embodiment, the flow path is a closed-circuit flow path, wherein the PFR comprises a tubing assembly packed in at least one chamber. In another aspect, the bypass line is an extension of the flow path that fluidically connects to the first and second locations within the flow path. The second location in the method can be positioned downstream from the first location, or the second location can be positioned upstream from the first location, or the second location can be positioned inside the PFR - downstream or upstream from the first location.

In another embodiment of the method, the pump is selected from a group consisting of a centrifugal pump and/or a positive displacement pump. In one aspect, the method further comprises a second pump in the flow path, wherein the second pump modulates the non-uniform feed stream flow in the bypass line. Alternatively, the method includes a second pump selected from the group consisting of: a centrifugal pump and/or a positive displacement pump.

In one embodiment, the non-uniform feed stream flow within the bypass line is co-current or counter-current to the non-uniform feed stream flow in the flow path. In one aspect, the bypass line diverts the non-uniform feed stream with a flow rate that is about 1% to about 99% of the fastest flow rate in the flow path.

In an embodiment according to claim 13 the invention is directed to a method of manufacturing a product of interest by a device as defined above. The invention is also directed to a method of manufacturing a protein by a device as defined above. In a preferred mode this protein is a therapeutic protein.

Additional features and advantages of various embodiments, aspects, will be set forth, in the description that follows, and will, be apparent from the description, or may be learned by the practice of various embodiments. The objectives and other advantages of various embodiments will be realized and attained using the elements and combinations particularly pointed out in the description herein.

### BRIEF DESCRIPTION OF THE DRAWINGS

The present disclosure, its several aspects, and embodiments can be more fully understood from the detailed description, claims, and the accompanying drawings.
Fig. 1 is a graph illustrating the relative concentration of protein eluate resulting from Protein A column chromatography as represented by an asymmetric Gaussian curve based on the inherent properties of the bind and elute process. The X-axis shows the Column Volume of the elution stream, and the Y-axis shows the Relative Concentration (%) of the eluate.
Fig. 2 illustrates an exemplary plurality of plug flow reactors.
Fig. 3 illustrates the flow pattern in each of the plug flow reactors shown in Fig. 2, according to an example of the present disclosure.
Fig. 4 illustrates a relationship between Protein A chromatography columns and the flow circulation device according to an example of the present disclosure;
Figs. 5(a) and 5(b) illustrate an example of a co-current flow circulation device with two plug flow reactors for homogenizing a non-uniform concentrated feed stream composition, according to an example of the present disclosure;
Fig. 6 illustrates an alternative co-current flow circulation device with six plug flow reactors for homogenizing a non-uniform concentrated feed stream composition, according to an example of the present disclosure;
Fig. 7 illustrates a counter-current flow circulation device with six plug flow reactors for homogenizing a non-uniform concentrated feed stream composition, according to an example of the present disclosure;
Fig. 8 illustrates the experimental results of circulating material around a simple non-PFR length of tubing to homogenize a composition;
Fig. 9 illustrates the washout volume of the simple non-PFR length of tubing, for which results are shown in Fig. 8;
Fig. 10 illustrates an experimental setup for a conventional in-line circulation;
Fig. 11 illustrates the results of the experimental setup of Fig. 10;
Fig. 12 illustrates the results of the experimental setup of Figs. 6, according to an example of the present disclosure;
Fig. 13 illustrates the pH and conductivity and UV per volume relating to the experimental setup shown in Figs. 5(a) and 5(b), according to an example of the present disclosure;
Fig. 14 illustrates the protein concentration after 30 minutes of circulation using the experimental setup shown in Figs. 5(a) and 5(b), according to an example of the present disclosure; and
Fig. 15 illustrates the washout volume required to wash out the Protein using the experimental setup shown in Figs. 5(a) and 5(b), according to an example of the present disclosure.

### DETAILED DESCRIPTION OF THE INVENTION

The following description is exemplary and intended to be nonlimiting to explain various embodiments of the present invention.

This disclosure is directed to a device and process for providing a thorough mixture, i.e., homogenization of a solution comprising a biotechnological product after one or more chromatographic purification steps.

So that the present invention may be more readily understood, the following terms are defined as follows. Additional definitions are also set forth throughout the detailed description.

### DEFINITIONS

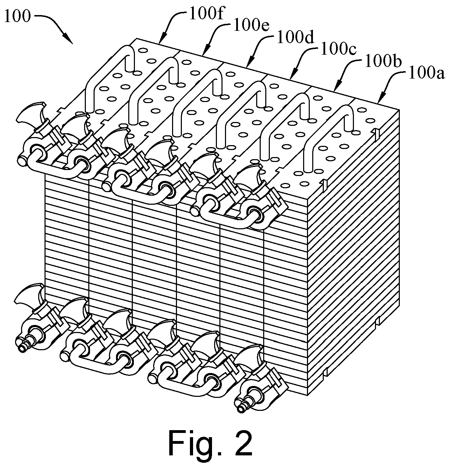

As used herein, the term "plug flow reactor (PFR)," also referred to as a "jig in a box (JIB)" or "circulation loop," refers to a tubing system or assembly (that forms a flow path) compressed in a 3-D space or chamber. An embodiment of a PFR can act as an elution stream chamber (ESC) or a tank-less hold, which is used between process steps wherein the output from a process that flows through the PFR to the next process step in a purification process. An embodiment of a PFR may include two or more chambers, as shown in Fig. 2. For example, in biopharmaceutical processing, different volumes of the elution stream flowing from at least one bind and elute (Protein A (ProA)) columns a PFR may have one more chambers fluidically connected to extend the flow path of a portion or the entire volume of the feed stream. A PFR may be used as a hold in between two process steps, e.g., between the bind and elute or ProA step and Virus Inactivation step. A PFR may also be a vessel used to provide residence time for the viral inaction step. A PFR also includes a bypass line. A PFR of the present invention also has a bypass line.

A "pump" is a device that moves fluids by mechanical action from one location, a first location, along the flow path to another location; a second location, along the flow path positionally different from the first location

As used herein, the term "Chamber" or "PFR chamber" refers to a section of a PFR's tubing assembly. A PFR chamber may include one or several loops or rows of tubing arranged in a continuous flow path, line, conduit, or tube of predetermined certain length and volume.

In an embodiment, the PFR holds or collects the entire volume of output from a process step and enables a continuous flow of output from one process step to the next (e.g., bind and elute or ProA step and Virus Inactivation step). In another embodiment, the volume of a PFR is about six times (6x, 6-fold, or 600%) the entire product containing volume of the output from a previous process step. In another embodiment, the volume of a PFR is about 500%, about 400%, about 300%, about 200%, about 150%, about 130%, about 120%, or about 110% of the entire product containing volume of the output from a previous process step. In alternative embodiments, the volume of a PFR is about 100% of the entire product-containing volume of the output from a previous process step. In another embodiment, the volume of a PFR is no more than 95%, 85%, 75%, 65%, 55%, 50% (half), 45%, 35%, 25%, 15%, 10% or 5% of the entire product containing volume of the output from a prior process step

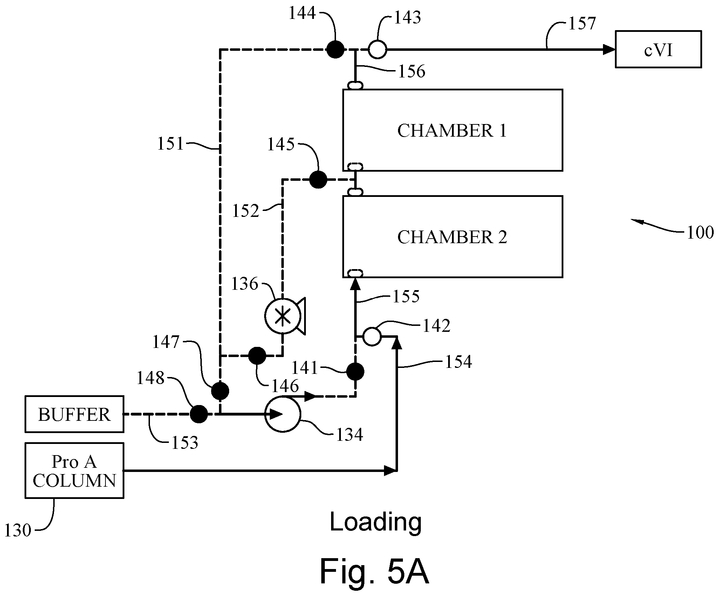

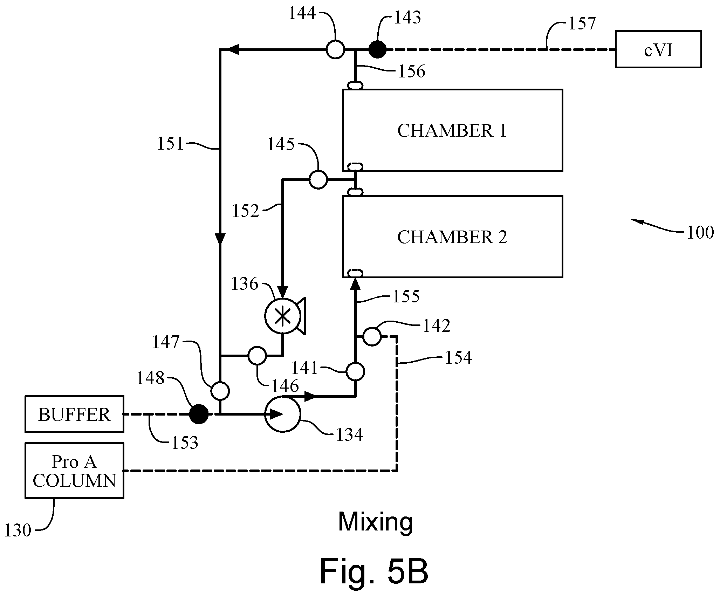

As used herein, the term "bypass line" in the context of a PFR refers to a tube or conduit that can divert about 1% to about 99% of the fastest fluid rate from a first location to a second location in a homogenization device of the present invention. See, for example, line 152 in Fig. 5(a) and 5 (b). The first location and the second location may be inside or outside of the PFR provided that one of the locations is inside the PFR. For example, the bypass line may divert 25% of the flow rate from a first location inside the PFR to a second location outside of the PFR within the flow path or tubing system that circulates the feed stream through the PFR. Alternatively, the bypass line may divert from about 1% to about 99%, or from about 10% to about 90%, or about 20% to 80%, or about 25% to 75 of a flow rate in the PFR from a first location to a second location. In an example, the bypass line is an extension of the PFR flow path that short-circuits the path between the first and second location by fluidically connecting two locations within the flow path. In an embodiment, the non-uniform feed stream flow in the bypass line operates in a co-current or counter-current direction to the non-uniform feed stream flow in the flow path. In another embodiment, the bypass line includes a pump that modulates the non-uniform feed stream flow into or through the bypass line in a co-current or counter-current direction to the non-uniform feed stream flow in the flow path or PFR.

As used herein, the term "substantially homogenized" term refers to a feed stream (e.g., bind and elute or ProA eluent stream) whose concentration profile has changed from a bell-shaped curve (e.g., Fig. 1) to an almost straight line. For example, a substantially homogenized feed stream may be a feed stream in which the feed stream concentration difference between the maximum and the minimum concentration contained within the PFR is reduced to a sufficient range. In another example, a substantially homogenized feed stream may be a feed stream whose peak maximum concentration (e.g., the non-uniform ProA elution) is dampened by from about 50% to about 99%. In an embodiment, a substantially homogenized feed stream has a concentration peak max dampened by about 50%, 60%, 70%, or about 80%, or about 90%, or about 95%, or about 99% compared to the concentration peak max of the same feed stream before the homogenization.

The term "in-line" or "in-line operation" refers to a process of moving a liquid sample through a tube or some other conduit without storage in a vessel, tank, or bag. Accordingly, in some embodiments, according to the present invention, a PFR is used in an "in-line operation" through which a liquid sample containing a target protein is moved from one process step to another.

The term "virus inactivation" or "V1" refers to the treatment of a sample potentially containing one or more viruses in a manner such one or more viruses are no longer able to replicate or are rendered inactive. Virus inactivation may be achieved by physical means, e.g., heat, ultraviolet light, ultrasonic vibration, or using chemical means, e.g., pH change or addition of a chemical virus inactivation reagent, and is a typical process step which is used during most protein purification processes, especially in case of purification of therapeutic proteins. In methods described herein, VI is performed using one or more in-line PFRs.

The term "virus inactivating agent" or "virus inactivation agent," refers to any physical or chemical means capable of rendering one or more viruses inactive or unable to replicate, A virus inactivating agent, as used by the methods described herein may include a solution condition change (e.g., pH, conductivity, temperature, etc.) or the addition of a solvent/detergent, a salt, a polymer, a small molecule, a drug molecule or any other suitable entity, for example, which interacts with one or more viruses in a sample or a physical means (e.g., exposure to UV light, vibration, etc.), such that exposure to the virus inactivating agent renders one or more viruses inactive or incapable of replicating. In a particular embodiment, a virus inactivation agent is a pH change, where the virus inactivating agent is mixed with a sample containing a target molecule (e.g., an eluate from a Protein A bind and elute chromatography step) using a PFR.

To reach the desired product-stream pH target as the feed stream elutes from the ProA column (given the product's inherent buffering capacity), two approaches could be implemented: (1) either the quantity of acid addition would have to change as a function of protein concentration, or (2) the fixed volumetric addition of acid would require to have enough strength to make the worst-case condition (i.e., peak maximum, where protein concentration is highest) reach the pH set point.

Alternatively or in addition, to avoid complexities associated with the product elution peak, such as variable viscosity and acid addition (i.e., the variable quantity of acid required to attain the pH set point as a function of protein concentration or fixed volume addition as a function of worst-case protein concentration), the peak concentration may be mixed such that the entire feed stream is of similar concentration and/or composition. Moreover, the feed stream should not be mixed in containers such as tanks or bags, as these vessels can't be easily sanitized, can't be completely emptied of protein elution from cycle to cycle, and have a probability of leaking the feed stream through a breach, a rupture from over pressurization, or any manner that potentially exposes the feed stream to the environment. Tanks and bags also have the complexity of containing air-liquid interfaces.

Accordingly, the device and method described herein avoids the complexities described above and homogenizes the feed stream significantly faster and more efficiently than existing systems: it can be easily sanitized, it has no air-liquid interfaces, and it is less likely to leak due to its rigid structure and design. Without wishing to be bound by any particular theory, the present invention is based, in part, on the unexpected finding that a non-uniform ProA elution feed stream (shown in Fig. 1) is effectively homogenized in-line and in a continuous and tank-less system by applying co-current and/or counter-current flows to the feed stream as it travels through one or more PFRs.

In an example, a plug flow reactor (PFR) 100 is shown in Fig. 2. The example PFR 100 shown in the figure includes six chambers **100a-100f.**

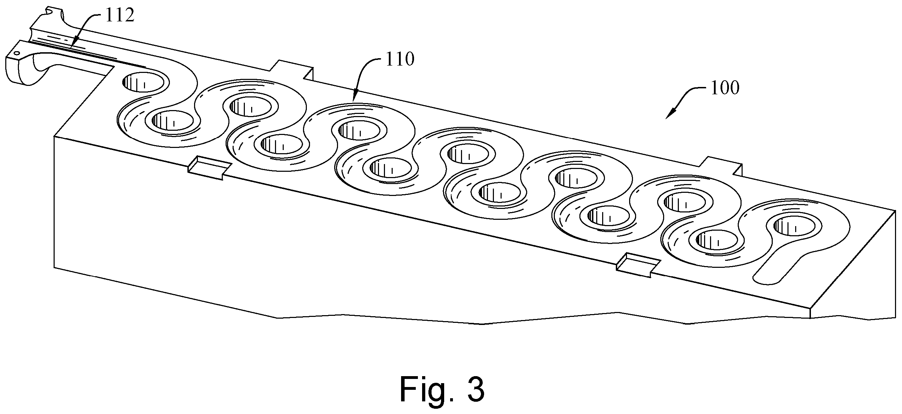

As shown in Fig. 3, each PFR 100 or a chamber thereof includes a tubular flow path 112 that includes turns or curves **110.** Details of similar PFRs are described in detail in PCT Application No. PCT/US2019/054959. As stated earlier, although the flow path is designed such that the direction of curvature from one turn to the next changes and/or shifts to enhance radial mixing and reduce axial dispersion, complete mixing is not achieved even after the feed stream is circuited through the PFR more than 17 times. See Fig. 11.

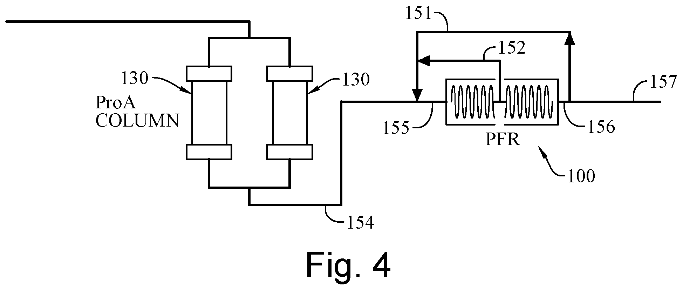

Referring to Fig. 4, in an example, the non-uniform feed stream composition is a ProA elution stream from ProA chromatography column(s) **130.** Generally, the total volume of the PFR **100** can be from about 1 to about 6 times the volume of the ProA chromatography column(s) **130,** excluding the volume inside the stream lines, such as fluidic lines **151, 152, 155** and **156** (also shown in Fig. 5). The volume of elution inside the fluidic lines **151, 152, 155** and **156** relative to the PFR **100** can be from about 10% to 0.5%, or about 7% to 3%, or a volume selected from the group consisting of about 20%, about 15%, about 10%, about 7%, about 5%, about 3%, about 1% and about 0.5%. In an embodiment, the volume of fluidic lines **151, 152, 155,** and **156** is about 3%, or about 2.2%, or about 1.6% of the total volume of the PFR 100, depending on the length of the fluidic lines.

Referring to Figs. 5(a) and 5(b), in an example, the device may also include at least a first pump **134,** an optional second pump **136** (which modulate the non-uniform feed stream flow in the bypass line **152),** a plurality of optional valves **141-148,** and a plurality of fluidic lines consecutively numbered **151-157.** In an embodiment, valves **141, 144, 145, 146 and/or 147** may be optional. In an example, each pair of valves **141** and **142, 143** and **144 or 147** and **148** may be combined and replaced with a three-way value. One of ordinary skill in the art appreciates that the position of values or pumps, or their number or types is not limiting as depicted and may be arranged differently to achieve a similar result of this disclosure. Initially, to load an example, PFR **100** with a non-uniform feed stream, e.g., the ProA elution stream, from column chromatography **130,** the first set of valves **142 and 143** are switched to an open position and the second set of valves **141, 144, 145, 146, 147, and 148** are switched to a closed position. As shown in Fig. 5(a), the ProA elution stream can then travel via feed stream line **154** and fluidic line **155** into the PFR **100** and displacing the internally held fluid out of fluidic lines **156** and **157.**

Referring to Fig. 5(b), once the ProA elution stream from column chromatography **130** is contained within the PFR **100,** the first set of valves **142 and 143** are switched to a closed position and a second set of valves **141, 144, 145, 146, 147 and 148** is switched to an open position to create a closed circuit loop or flow path such that there is no inlet and outlet to the flow path. Each of the first and second pumps **134** and **136** may then circulate the ProA elution stream several times via fluidic lines **151, 152, 155, and 156** such that the ProA elution stream is homogenized (*i.e.,* forming a uniform concentrated feed stream composition) by mixing at the union of stream line **151 and 152.**

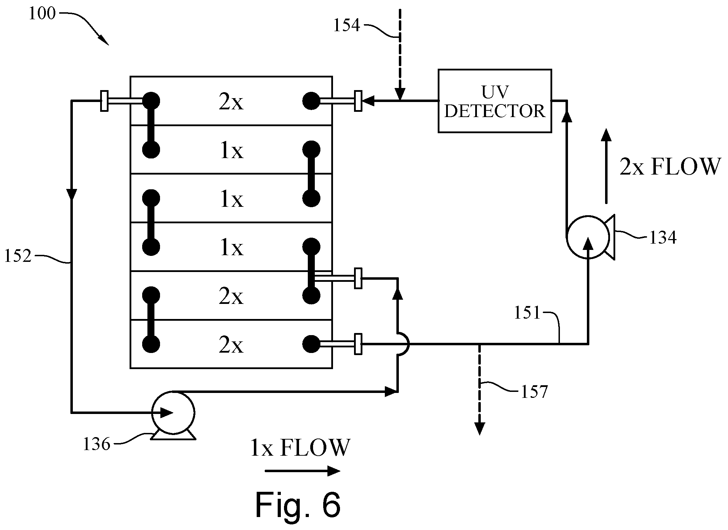

In an example, as shown in Figs. 5 and 6, the device homogenizes the non-uniform feed stream (e.g., the ProA elution stream) by a co-current circulation. A co-current circulation is achieved by a bypass line *(i.e.,* stream line **152)** diverting a predefined percentage of the circulating feed stream (defined as a percent feed rate) from a first location in the flow path to a second location downstream of the first location within the closed-circuit flow path such that the result is a decreased flow rate in a portion of the stream lines.

Referring to Fig. 5(b), in an example, the PFR **100** includes two chambers in fluid communication with one another. As discussed earlier, the size of a PFR depends on the volume of the Protein A elution stream. In this example, once the first set of valves **142 and 143** are closed and the second set of valves **141, 144, 145, 146, 147 and 148** are opened, the first pump **134** pushes the ProA elution stream through the line **155** towards Chamber 2 of the PFR **100.** A bypass line **152** diverts -from about 30% to about 90%, such as from about 50% to about 80%, for example, about 75% of- the ProA elution stream from a first location in the PFR to a second location within the closed-circuit flow path which includes the PFR and lines **151, 155 and 156.** The remaining ProA elution stream continues to flow into Chamber 1 of PFR **100,** which can then exit the PFR **100** via stream lines **156** and **151** towards the first pump **134.** Before reaching the first pump **134,** the bypass line **151** merges with the stream line **152** and the ProA elution in each of the two stream lines **151** and **152** mixes. The first pump **134** then guides the combined ProA elution stream through line **155** into the PFR **100** again for yet another circulation round.

In an embodiment, the first pump **134** and the second pump **136** have different flow rates. For example, the flow rate of the second pump **136** is less than the flow rate of the first pump **134.** In another embodiment, the flow rate of the second pump **136** which is defined by being the pump which operates at a flow rate less than the flow rate of the first pump **134** s such that it can divert from about 1% to about 99% of the total flow rates in the reactors (i.e., in this particular example, the total going through the first pump **134),** such as from about 10% to about 90% of the total flow rates in the reactors, or from about 50% to about 80%, such as a flow rate of about 75% of the total flow rate in the reactors.

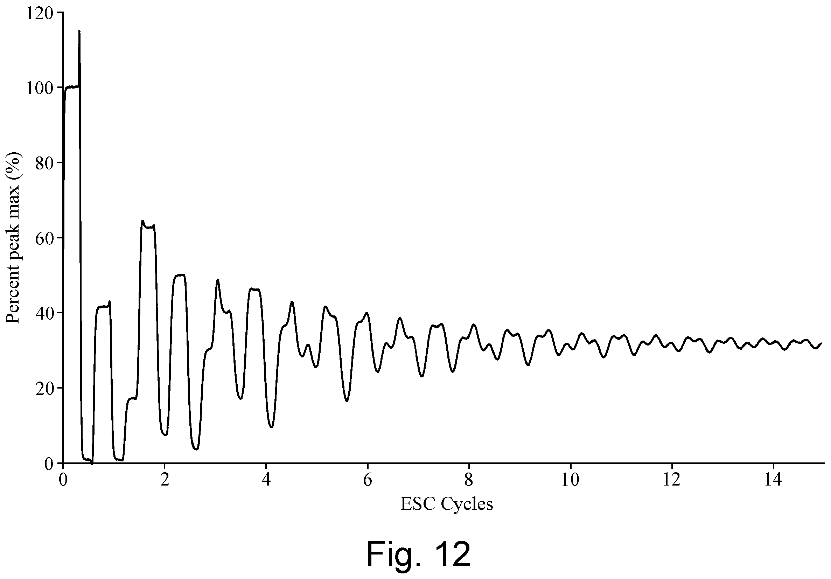

In an embodiment, a PFR **100** may be arranged such that, when a non-uniform concentrated feed stream such as that shown in Fig. 1 is introduced into the PFR **100** and circulated a predetermined number of times in the closed-circuit flow path represented by fluidic lines **155, 156, 151, and 152** and pump **134** such that the non-uniform concentrated feed stream becomes substantially homogenized. See Fig. 12. In an embodiment, the nonuniform concentrated feed stream is fed through the PFR of the present invention and becomes homogenized by at least 20%, 25%, 30%, 35%, 40%, 50%, 60%, 70%, 80%, 90% or 100%.

In an example, in Fig. 6, a co-current circulating system for homogenizing a non-uniform feed stream (e.g., ProA elution feed stream) is shown. The co-current circulation method or device may be characterized by a PFR in which a bypass line **152** diverts a percentage of the fluid flow from a first location in the PFR to a second location which is downstream of the first location such that the result is a decreased flow rate in a portion of the stream lines. As shown, the first pump **134** drives the feed stream through line **151** into the PFR **100.** With the assistance of second pump **136,** the bypass line **152** diverts approximately 50% of the flow rate from a first location (e.g., first chamber) to a second location (e.g., fifth chamber) downstream of the first location in the PFR **100.** Given that 50% of the feed stream was diverted from the first chamber in the PFR **100,** the second, the third, and the fourth chambers contain only 50% of the flow rate as represented by "1x". The diverted feed stream and the non-diverted feed stream merge in the fifth chamber in PFR **100.** The combined feed stream then flows to the sixth chamber, where the bypass line **152** again diverts the combined feed stream from chamber 1 to chamber 5 and thus repeating the above flow pattern once again. In this example, the flow rate of the first pump **134** is approximately 2 times the flow rate of the second pump **136.**

The co-current circulation process may be repeated for a sufficient number of times, such that the maximum peak concentration of the non-uniform ProA elution is dampened from about 70% to about 90%. For example, the circulation process can repeat from about 3 times to about 20 times, such as from about 5 times to about 10 times, for example, 6 times. Alternatively, the circulation process can repeat for a predetermined amount of time, such as from about 10 minutes to about 3 hours, for example, for about 30 minutes.

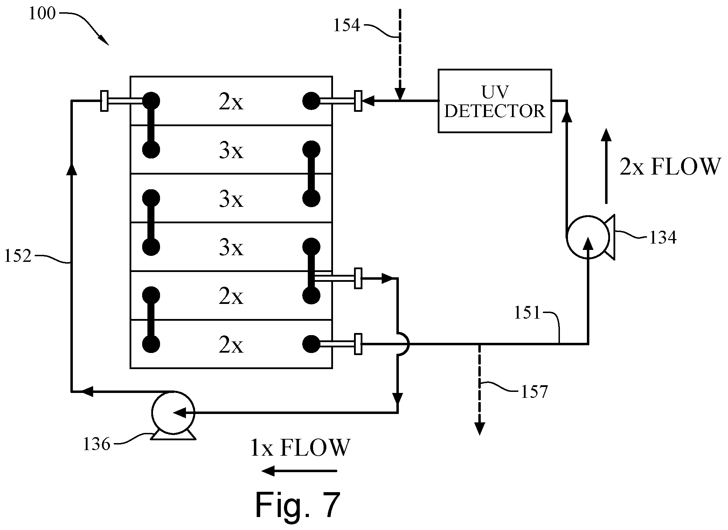

In another example, as shown in Fig. 7, a counter-current circulating system for homogenizing a non-uniform feed stream (e.g., ProA elution feed stream) is shown. The counter-current circulation method or device may be characterized by a PFR in which a bypass line diverts a percentage of the fluid flow from a first location in the PFR to a second location which is upstream of the first location such that the result is an increased flow rate in a portion of the stream lines. As shown, in a counter-current example, the flow rate of the second pump **136** is defined by being the pump that operates at a flow rate less than or equal to the flow rate of the first pump **134.** With the assistance of pump **136,** the bypass line **152** diverts anywhere from about 1% to about 100% of the total flow rate from a first location (e.g., chamber 4) to a second location (e.g., chamber 1) in the PFR **100.** In an embodiment, the bypass line diverts from about 10% to about 90% of the total flow rate, or from about 50% to about 80%, or about 40%, or about 50%, or about 60%, or about 75% of the flow rate from a first location to a second location upstream of the first location in the PFR. The combined feed stream then flows through the PFR in chambers two through 4, where the bypass line **152** again diverts the combined feed stream from chamber 4, repeating the above flow pattern once again.

The counter-current circulation process may be repeated for a sufficient number of times, such that the maximum peak concentration of the non-uniform ProA elution is dampened by from about 70% to about 90%. For example, the circulation process can repeat from about 3 times to about 20 times, such as from about 5 times to about 10 times, for example, 6 times. Alternatively, the circulation process can repeat for a predetermined amount of time, such as from about 10 minutes to about 3 hours, for example, for about 30 minutes.

Alternatively or in addition, Fig. 7 illustrates a counter-current circulation device for homogenizing a ProA elution stream exiting a column chromatography. In this example, the PFR **100** has six chambers. The first pump **134** drives the ProA elution stream into the PFR **100** at chamber one via stream line **151.** The ProA elution stream then travels from chamber 1 to chamber 4 where the bypass line **152** (with the help of the second pump **136)** diverts 1/3 (or 1X) of the maximum flow rate of the ProA elution stream from there to the second chamber to create a combined (or 3X) ProA elution stream. The combined (3X) ProA elution stream then flows into the second, third, and fourth chambers of the PFR **100.** At the end of the fourth chamber of the PFR **100,** the bypass line **152** again diverts (1X) of the elution stream back to chamber 2, and the same flow pattern repeats itself. This process repeats for a sufficient number of times, such that the maximum peak concentration of the non-uniform ProA elution is dampened by from about 70% to about 90%. For example, the circulation process can repeat from about 3 times to about 20 times, such as from about 5 times to about 10 times, for example, 6 times. Alternatively, the circulation process can repeat for a predetermined amount of time, such as from about 10 minutes to about 3 hours, for example, for about 30 minutes. In this example, the flow rate of the first pump **134** is approximately 2 times the flow rate of the second pump **136.**

In each of the co-current and counter-current circulation devices, either pump can be any combination of centrifugal and positive displacement pumps such that they both are positive displacement pump, both are centrifugal pumps, or one is centrifugal and one positive displacement. More preferably, the first pump **134** (*i.e.,* faster-operating pump) may be a centrifugal pump and the second pump **136** (*i.e.,* slower operating pump) may be a positive displacement pump. These pumps govern the fluid dynamics of the design (*e.g*., prevention of pressure and pump deadheading issues). Furthermore, each of the co-current and counter-current circulation devices may include a loading flow rate of at least 200 mL/min and an output flow rate into an incubation chamber of about 43 mL/min or more. Moreover, each of the co-current and counter-current circulation devices may include a loop volume of at least 3 times the volume of the chromatography column and include a maximum flow rate of about 3.5 times or more of the elution flow (e.g., a flow rate of about 700 mL/min or more) or 16.3 times the flow rate relative to loop chase.

### Examples

### Example 1

### Homogenizing a non-uniform feed stream using a tubing system

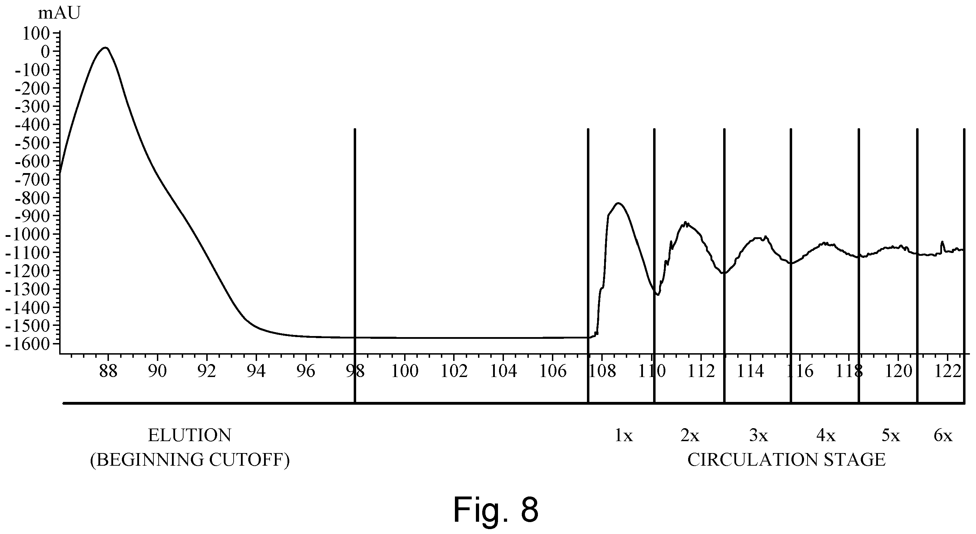

To homogenize a non-uniform feed stream, one solution would be to circulate material around a length of tubing to homogenize the composition. Mechanistically, this occurs due to the axial dispersion experienced by the flowing liquid in a tube. Fig. 8 illustrates an experiment in which protein was eluted into a length of tubing and circulated. Once the protein was circulated about 6 times, the UV A280 trace, which translates to protein concentration, becomes relatively flat. This change is indicative of complete mixing.

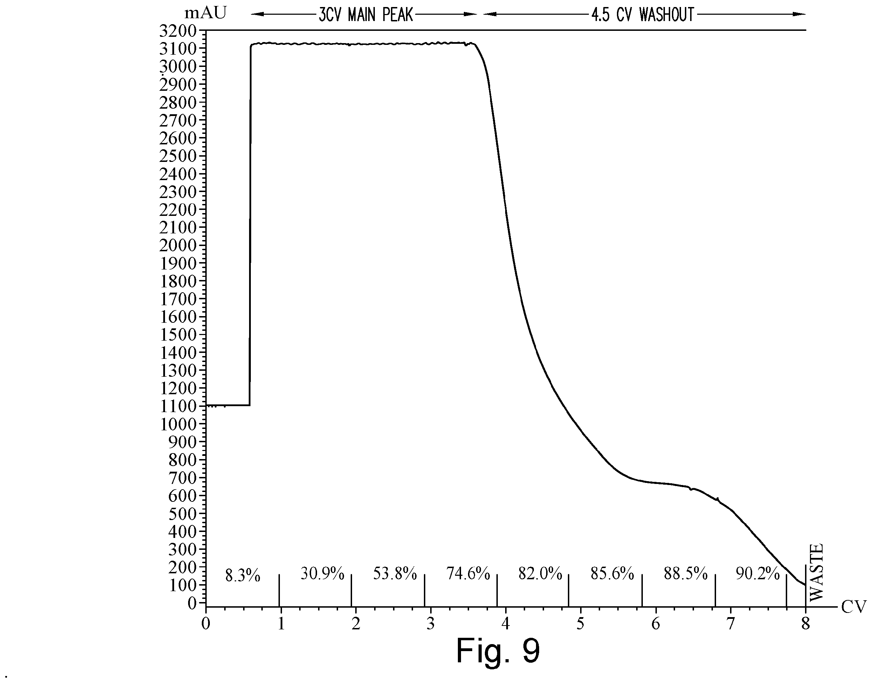

Once the mixing is completed, the mixed protein required a push out of the tubing and to the next unit operation in-line without significant dispersion of the contents. Due to the same mechanism that caused the mixing to occur (*i.e.,* axial dispersion), significant product tailing occurred-this ultimately led to a loss in time and product. Fig. 9 shows the washout of the mixing loop, where CV is column volume relative to that of the eluting Protein A (ProA) column. Also, this tailing added complexity for sanitizing and rinsing the device since the quantity required would exceed 7.5 CV-further increasing the time and volume needed for the step.

### Example 2

### Homogenizing a non-uniform feed stream using a PFR system As described in PCT/US2019/054959

Materials: The PFR is characterized by its repeating serpentine flow paths. The PFR was 3D printed using stereolithography (SLA) technology at 3D Systems (Rock Hill, SC). The riboflavin used in creating the mobile phases and pulse tracer was purchased through Thermo Fisher Scientific (Suwanee, GA).

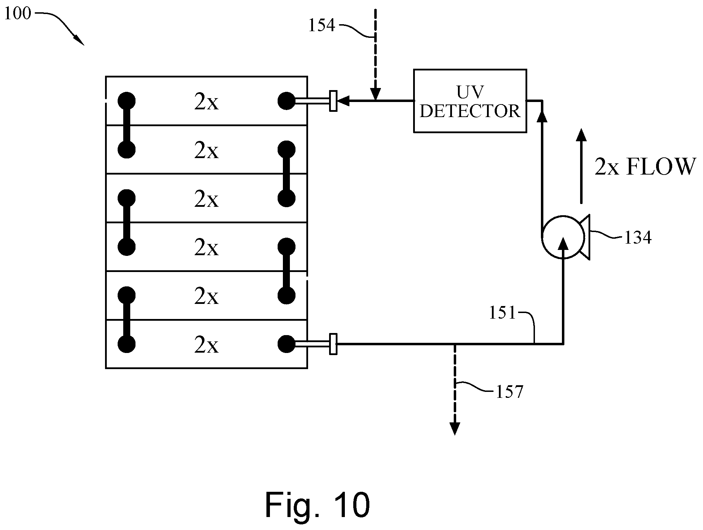

To homogenize a non-uniform feed stream, another solution may be to use A PFR system such that shown in Fig. 10. The PFR was designed to disrupt axial dispersion by generating radial mixing through Dean vortices. This radial mixing is perfect for allowing the vessel to be filled and washed out to minimize time and volume requirements, but far from optimal for circulation. To confirm that a simple solution would not work, an experiment was conducted where 1 L of riboflavin was inserted into a 3 L PFR and circulated. Fig. 10 illustrates the experimental setup.

As illustrated in Fig. 11, after approximately 17 passes, peak max was only dampened by about 10%.

### Example 3

### Homogenizing a non-uniform feed stream using a PFR system of the present invention

In an attempt to invent a more effective means of mixing the peak in the PFR while maintaining the ability to empty the PFR, the two designs illustrated in Figs. 6 and 7 were created.

The co-current design (Fig. 6) is characterized by the creation of a bypass line to divert a fraction of the circulating liquid for displacement farther downstream, such that the result is a decreased flow rate in a portion of the stream lines. The counter-current design (Fig. 7) is characterized by the creation of a return line to divert a fraction of the circulating liquid for displacement farther upstream, such that the result is an increased flow rate in a portion of the stream lines. Fig. 12 illustrates the result of the experiment conducted using the co-current method. The variables tested in these designs included the following: location within the closed-loop of the bypass/return line, changes/alternations between co-current and counter-current methods during the experiment, and flow rate ratio of the pumps.

The co-current design, on the other hand, generated less pressure. As for the spacing of the bypass line, equal volume distance of the bypass line was chosen since this would require two identical PFRs, as seen in Figs. 5(a) and 5(b). The faster-operating pump was a centrifugal pump and is called the primary circulating pump (PCP). The slower operating pump was a positive displacement pump called the secondary mixing pump (SMP). The selection and placement of these pumps are critical to the fluid dynamics of the design (e.g., prevention of pressure and pump deadheading issues).

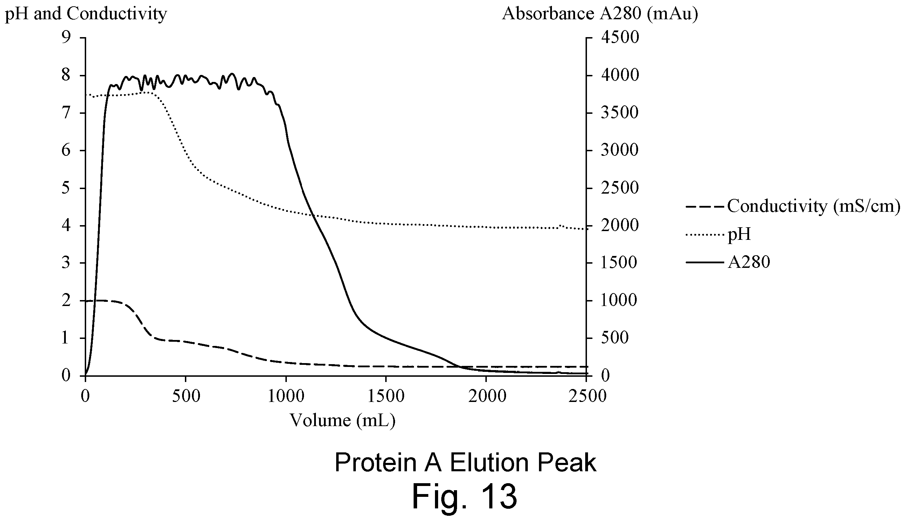

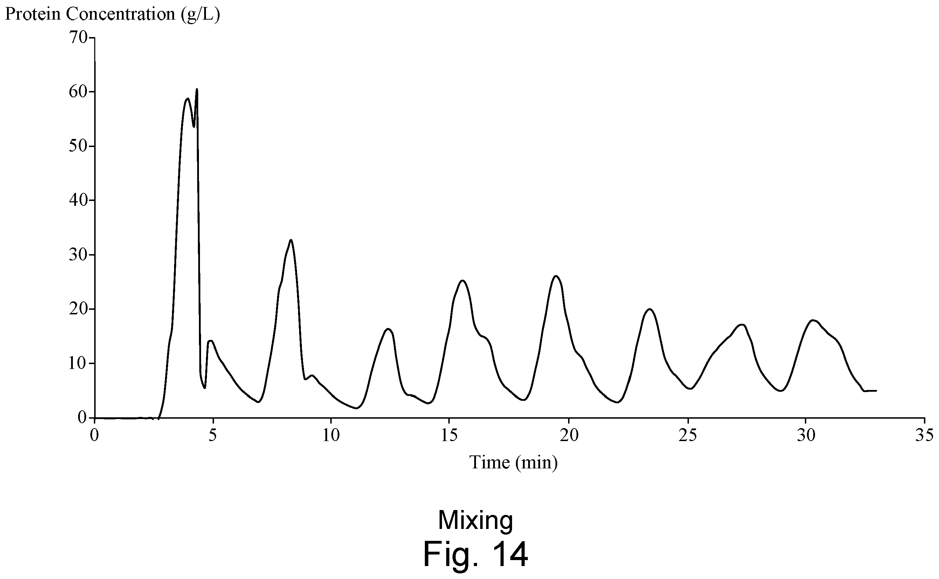

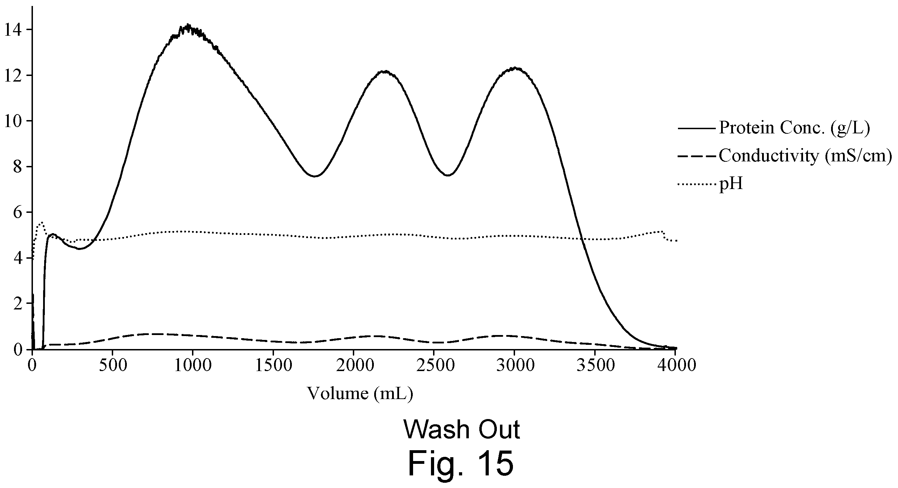

Testing the design shown in Figs. 5(a) and 5(b) with Protein, Figs. 13, 14, and 15 show traces from the elution peak sourced from a ProA column, the mixing within the reactors, and the subsequent washout, respectively. The ProA elution curve shown in Fig. 14 is sourced from a 1 L ProA column. The peak max of the elution peak had a high enough protein concentration such that the UV detector of the chromatography skid became saturated, and the A280 curve appears flat maxing out at ~4000 mAu. However, during the mixing portion of the operation, shown in Fig. 14, a variable path length UV detector (FlowVPE) was used and therefore was not susceptible to saturation, so it was able to capture the 60 g/L peak max before mixing.

The key takeaway points from this set of experimental data are that a protein peak maximum of ~60 g/L was dampened to 8-14 g/L and that the subsequent protein washout took 3.8 CVs.

## Claims

1. A device for in-line homogenization of a non-uniform feed stream, said device comprising a flow path comprising:
(a) a plug flow reactor (PFR; 100);
(b) a pump (134) for circulating the non-uniform feed stream through the flow path, **characterized by**
(c) a bypass line (152) for diverting a percentage of the non-uniform feed stream from a first location inside the PFR (100) to a second location within the flow path.

2. The device of claim 1, wherein the non-uniform feed stream comprises a protein or product of interest.

3. The device of claim 1 or 2, wherein the device is in-line between a first and a second process step, the first process is preferably selected from the group consisting of: bind and elute chromatography and Protein A chromatography and/or
the second process step is preferably a virus inactivation step.

4. The device of any of the preceding claims 1 to 3, comprising one, two, three or more of the following features:
- the device is for virus inactivation;
- the flow path is a closed-circuit flow path;
- the device is tank-less;
- the PFR (100) comprises a tubing assembly packed in at least one chamber;
- the bypass line (152) is an extension of the flow path that fluidically connects to locations within the flow path;
- the second location is downstream from the first location;
- the second location is upstream from the first location;
- the second location is inside the PFR (100) downstream or upstream from the first location;
- the pump is a pump selected from the group consisting of: a centrifugal pump and a positive displacement pump;and/or
- the non-uniform feed stream flow in the bypass line (152) is co-current or counter-current to the non-uniform feed stream flow in the flow path.

5. The device of any of the preceding claims 1 to 4, wherein the device further comprises a second pump (136) in the flow path, wherein the second pump (136) preferably modulates the non-uniform feed stream flow in the bypass line (152), and/or
the second pump (136) is preferably a pump selected from the group consisting of a centrifugal pump and a positive displacement pump.

6. The device of claim 5, wherein the non-uniform feed stream flow in the bypass line (152) is co-current or counter-current to the non-uniform feed stream flow in the flow path.

7. A method for in-line homogenizing a non-uniform feed stream, comprising:
(a) Providing a non-uniform feed stream,
(b) Flowing the non-uniform feed stream through a flow path comprising a PFR (100), a bypass line (152) and a pump (134);
(c) Diverting a percentage of the non-uniform feed stream from a first location inside the PFR (100) to a second location within the flow path;
(d) Circulating the non-uniform feed stream through the flow path; and
(e) Homogenizing the non-uniform feed stream.

8. The method of claim 7, comprising one, two, three or more of the following features:
- the non-uniform feed stream comprises a protein or product of interest;
- the method is for virus inactivation;
- the flow path is a closed-circuit flow path;
- the PFR (100) comprises a tubing assembly packed in at least one chamber;
- the bypass line (152) is an extension of the flow path that fluidically connects to the first and second locations within the flow path.
- the second location is downstream from the first location;
- the second location is upstream from the first location;
- the second location is inside the PFR (100) downstream or upstream from the first location; and/or
- the pump (134) is a pump selected from the group consisting of a centrifugal pump and a positive displacement pump.

9. The method of any of the preceding claims 8 or 9, wherein the flow path is in-line between a first and a second process step,, wherein the first process step is preferably a Protein A (ProA) column chromatograph and/or the second process step is preferably a virus inactivation step.

10. The method of any of the preceding claims 7 to 9, wherein the method further comprises a second pump (136) in the flow path, wherein the second pump (136) preferably modulates the non-uniform feed stream flow in the bypass line (152) and/or the second pump (136) is preferably a pump selected from the group consisting of a centrifugal pump and a positive displacement pump.

11. The method of any of the preceding claims 7 to 10, wherein the non-uniform feed stream flow in the bypass line (152) is co-current or counter-current to the non-uniform feed stream flow in the flow path.

12. The method of claim 11, wherein the bypass line (152) diverts the non-uniform feed stream with a flow rate that is about 1% to 99% of the flow rate in the flow path.

13. Method of manufacturing a product of interest by a device according to any of the preceding claims 1 to 6.

14. Method of claim 13 wherein the product of interest is a protein, preferably a therapeutic protein.

## Patentansprüche

1. Vorrichtung zur Inline-Homogenisierung eines ungleichmäßigen Zufuhrstroms, wobei die Vorrichtung einen Strömungsweg aufweist, umfassend:
(a) einen Plug-Flow-Reaktor (PFR; 100);
(b) eine Pumpe (134) zum Zirkulieren des ungleichmäßigen Zufuhrstroms durch den Strömungsweg,
**gekennzeichnet durch**
(c) eine Bypassleitung (152) zum Umleiten eines Prozentsatzes des ungleichmäßigen Zufuhrstroms von einer ersten Stelle innerhalb des PFR (100) zu einer zweiten Stelle innerhalb des Strömungswegs.

2. Vorrichtung nach Anspruch 1, wobei der ungleichmäßige Zufuhrstrom ein Protein oder ein Produkt von Interesse umfasst.

3. Vorrichtung nach Anspruch 1 oder 2, wobei die Vorrichtung zwischen einem ersten und einem zweiten Verfahrensschritt in Reihe geschaltet ist, wobei der erste Verfahrensschritt vorzugsweise ausgewählt ist aus der Gruppe, bestehend aus: Bindungs- und Elutionschromatographie und Protein-A-Chromatographie und/oder
der zweite Verfahrensschritt vorzugsweise einen Virusinaktivierungsschritt darstellt.

4. Vorrichtung nach irgendeinem der vorangehenden Ansprüche 1 bis 3, umfassend ein, zwei, drei oder mehr der folgenden Merkmale:
- die Vorrichtung dient zur Inaktivierung von Viren;
- der Strömungsweg ist ein geschlossener Strömungsweg;
- die Vorrichtung ist tanklos;
- der PFR (100) umfasst eine in mindestens eine Kammer gepackte Schlauchanordnung;
- die Bypassleitung (152) ist eine Verlängerung des Strömungswegs, die fluidmäßig mit Stellen innerhalb des Strömungswegs verbunden ist;
- die zweite Stelle liegt stromabwärts von der ersten Stelle;
- die zweite Stelle liegt stromaufwärts von der ersten Stelle;
- die zweite Stelle liegt innerhalb des PFR (100) stromabwärts oder stromaufwärts von der ersten Stelle;
- die Pumpe ist eine Pumpe, ausgewählt aus der Gruppe, bestehend aus einer Zentrifugalpumpe und einer Verdrängungspumpe; und/oder
- der ungleichmäßige Zufuhrstrom in der Bypassleitung (152) fließt gleichläufig oder gegenläufig zum ungleichmäßigen Zufuhrstrom im Strömungsweg.

5. Vorrichtung nach irgendeinem der vorangehenden Ansprüche 1 bis 4, wobei die Vorrichtung weiterhin eine zweite Pumpe (136) im Strömungsweg umfasst, wobei die zweite Pumpe (136) vorzugsweise den ungleichmäßigen Zufuhrstrom in der Bypassleitung (152) moduliert und/oder
die zweite Pumpe (136) vorzugsweise eine Pumpe ist, ausgewählt aus der Gruppe, bestehend aus einer Zentrifugalpumpe und einer Verdrängungspumpe.

6. Vorrichtung nach Anspruch 5, wobei der ungleichmäßige Zufuhrstrom in der Bypassleitung (152) gleichläufig oder gegenläufig zum ungleichmäßigen Zufuhrstrom im Strömungsweg ist.

7. Verfahren zum Inline-Homogenisieren eines ungleichmäßigen Zufuhrstroms, umfassend:
(a) Bereitstellen eines ungleichmäßigen Zufuhrstroms,
(b) Leiten des ungleichmäßigen Zufuhrstroms durch einen Strömungsweg, umfassend einen PFR (100), eine Bypassleitung (152) und eine Pumpe (134);
(c) Umleiten eines Prozentsatzes des ungleichmäßigen Zufuhrstroms von einer ersten Stelle innerhalb des PFR (100) zu einer zweiten Stelle innerhalb des Strömungswegs;
(d) Zirkulieren des ungleichmäßigen Zufuhrstroms durch den Strömungsweg; und
(e) Homogenisieren des ungleichmäßigen Zufuhrstroms.

8. Verfahren nach Anspruch 7, umfassend ein, zwei, drei oder mehr der folgenden Merkmale:
- der ungleichmäßige Zufuhrstrom umfasst ein Protein oder ein Produkt von Interesse;
- das Verfahren dient zur Virusinaktivierung;
- der Strömungsweg ist ein geschlossener Strömungsweg;
- der PFR (100) umfasst eine in mindestens eine Kammer gepackte Schlauchanordnung;
- die Bypassleitung (152) ist eine Verlängerung des Strömungswegs, die fluidmäßig mit der ersten und der zweiten Stelle innerhalb des Strömungswegs verbunden ist.
- die zweite Stelle liegt stromabwärts von der ersten Stelle;
- die zweite Stelle liegt stromaufwärts von der ersten Stelle;
- die zweite Stelle liegt innerhalb des PFR (100) stromabwärts oder stromaufwärts von der ersten Stelle; und/oder
- die Pumpe (134) ist eine Pumpe, ausgewählt aus der Gruppe, bestehend aus einer Zentrifugalpumpe und einer Verdrängungspumpe.

9. Verfahren nach irgendeinem der vorangehenden Ansprüche 8 oder 9, wobei der Strömungsweg zwischen einem ersten und einem zweiten Verfahrensschritt in Reihe angeordnet ist, wobei der erste Verfahrensschritt vorzugsweise eine Protein-A-(ProA)-Säulenchromatographie ist und/oder der zweite Verfahrensschritt vorzugsweise einen Virusinaktivierungsschritt darstellt.

10. Verfahren nach irgendeinem der vorangehenden Ansprüche 7 bis 9, wobei das Verfahren weiterhin eine zweite Pumpe (136) in dem Strömungsweg umfasst, wobei die zweite Pumpe (136) vorzugsweise den ungleichmäßigen Zufuhrstrom in der Bypassleitung (152) moduliert und/oder die zweite Pumpe (136) vorzugsweise eine Pumpe ist, ausgewählt aus der Gruppe, bestehend aus einer Zentrifugalpumpe und einer Verdrängungspumpe.

11. Verfahren nach irgendeinem der vorangehenden Ansprüche 7 bis 10, wobei der ungleichmäßige Zufuhrstrom in der Bypassleitung (152) gleichläufig oder gegenläufig zum ungleichmäßigen Zufuhrstrom im Strömungsweg ist.

12. Verfahren nach Anspruch 11, wobei die Bypassleitung (152) den ungleichmäßigen Zufuhrstrom mit einer Durchflussrate umleitet, die etwa 1 % bis 99 % der Durchflussrate im Strömungsweg beträgt.

13. Verfahren zur Herstellung eines Produkts von Interesse durch eine Vorrichtung nach irgendeinem der vorangehenden Ansprüche 1 bis 6.

14. Verfahren nach Anspruch 13, wobei das Produkt von Interesse ein Protein, vorzugsweise ein therapeutisches Protein, darstellt.

## Revendications

1. Dispositif pour l'homogénéisation en ligne d'un flux d'alimentation non uniforme, ledit dispositif comprenant un trajet d'écoulement comprenant :
(a) un réacteur à écoulement piston (PFR ; 100) ;
(b) une pompe (134) pour faire circuler le flux d'alimentation non uniforme sur le trajet d'écoulement, **caractérisée par**
(c) une conduite de dérivation (152) pour dévier un pourcentage du flux d'alimentation non uniforme d'un premier emplacement dans le PFR (100) vers un second emplacement dans le trajet d'écoulement.

2. Dispositif selon la revendication 1, dans lequel le flux d'alimentation non uniforme comprend une protéine ou un produit d'intérêt.

3. Dispositif selon la revendication 1 ou 2, dans lequel le dispositif est en ligne entre une première et une seconde étape de procédé, le premier procédé est de préférence sélectionné dans le groupe consistant en : la chromatographie de liaison et d'élution et la chromatographie d'affinité sur protéine A et/ou
la seconde étape de procédé est de préférence une étape d'inactivation virale.

4. Dispositif selon l'une quelconque des revendications 1 à 3 précédentes, comprenant une, deux, trois ou plus des caractéristiques suivantes :
- le dispositif est destiné à l'inactivation virale ;
- le trajet d'écoulement est un trajet d'écoulement en circuit fermé ;
- le dispositif est sans réservoir ;
- le PFR (100) comprend un ensemble tubulaire enveloppé par au moins une chambre ;
- la conduite de dérivation (152) est une extension du trajet d'écoulement qui est reliée fluidiquement à des emplacements dans le trajet d'écoulement ;
- le second emplacement est en aval du premier emplacement ;
- le second emplacement est en amont du premier emplacement ;
- le second emplacement est dans le PFR (100) en aval ou en amont du premier emplacement ;
- la pompe est une pompe sélectionnée dans le groupe consistant en : une pompe centrifuge et une pompe volumétrique ; et/ou
- l'écoulement de flux d'alimentation non uniforme dans la conduite de dérivation (152) est parallèle ou à contre-courant par rapport à l'écoulement de flux d'alimentation non uniforme dans le trajet d'écoulement.

5. Dispositif selon l'une quelconque des revendications 1 à 4 précédentes, dans lequel le dispositif comprend en outre une seconde pompe (136) dans le trajet d'écoulement, dans lequel la seconde pompe (136) module de préférence l'écoulement de flux d'alimentation non uniforme dans la conduite de dérivation (152), et/ou
la seconde pompe (136) est de préférence une pompe sélectionnée dans le groupe consistant en une pompe centrifuge et une pompe volumétrique.

6. Dispositif selon la revendication 5, dans lequel l'écoulement de flux d'alimentation non uniforme dans la conduite de dérivation (152) est parallèle ou à contre-courant par rapport à l'écoulement de flux d'alimentation non uniforme dans le trajet d'écoulement.

7. Méthode pour l'homogénéisation en ligne d'un flux d'alimentation non uniforme, comprenant :
(a) la fourniture d'un flux d'alimentation non uniforme,
(b) l'écoulement du flux d'alimentation non uniforme sur un trajet d'écoulement comprenant un PFR (100), une conduite de dérivation (152) et une pompe (134) ;
(c) la déviation d'un pourcentage du flux d'alimentation non uniforme d'un premier emplacement dans le PFR (100) vers un second emplacement dans le trajet d'écoulement ;
(d) la circulation du flux d'alimentation non uniforme sur le trajet d'écoulement ; et
(e) l'homogénéisation du flux d'alimentation non uniforme.

8. Méthode selon la revendication 7, comprenant une, deux, trois ou plus des caractéristiques suivantes :
- le flux d'alimentation non uniforme comprend une protéine ou un produit d'intérêt ;
- la méthode est destinée à l'inactivation virale ;
- le trajet d'écoulement est un trajet d'écoulement en circuit fermé ;
- le PFR (100) comprend un ensemble tubulaire enveloppé par au moins une chambre ;
- la conduite de dérivation (152) est une extension du trajet d'écoulement qui est reliée fluidiquement aux premier et second emplacements dans le trajet d'écoulement ;
- le second emplacement est en aval du premier emplacement ;
- le second emplacement est en amont du premier emplacement ;
- le second emplacement est dans le PFR (100) en aval ou en amont du premier emplacement ; et/ou
- la pompe (134) est une pompe sélectionnée dans le groupe consistant en une pompe centrifuge et une pompe volumétrique.

9. Méthode selon l'une quelconque des revendications 8 ou 9 précédentes, dans laquelle le trajet d'écoulement est en ligne entre une première et une seconde étape de procédé, dans laquelle la première étape de procédé est de préférence un chromatographe sur colonne pour la chromatographie d'affinité sur protéine A (ProA) et/ou la seconde étape de procédé est de préférence une étape d'inactivation virale.

10. Méthode selon l'une quelconque des revendications 7 à 9 précédentes, dans laquelle la méthode comprend en outre une seconde pompe (136) dans le trajet d'écoulement, dans laquelle la seconde pompe (136) module de préférence l'écoulement de flux d'alimentation non uniforme dans la conduite de dérivation (152) et/ou la seconde pompe (136) est de préférence une pompe sélectionnée dans le groupe consistant en une pompe centrifuge et une pompe volumétrique.

11. Méthode selon l'une quelconque des revendications 7 à 10 précédentes, dans laquelle l'écoulement de flux d'alimentation non uniforme dans la conduite de dérivation (152) est parallèle ou à contre-courant par rapport à l'écoulement de flux d'alimentation non uniforme dans le trajet d'écoulement.

12. Méthode selon la revendication 11, dans laquelle la conduite de dérivation (152) dévie le flux d'alimentation non uniforme avec un débit qui est d'environ 1 % à 99 % du débit dans le trajet d'écoulement.

13. Méthode de fabrication d'un produit d'intérêt par un dispositif selon l'une quelconque des revendications 1 à 6 précédentes.

14. Méthode selon la revendication 13 dans laquelle le produit d'intérêt est une protéine, de préférence une protéine thérapeutique.
